(19) Europäisches Patentamt
European Patent Office
Office européen des brevets

(11) Veröffentlichungsnummer: **0 009 852**
**B1**

(12) # EUROPÄISCHE PATENTSCHRIFT

(45) Veröffentlichungstag der Patentschrift: **22.09.82**

(51) Int. Cl.³: **C 12 M 1/08**, C 12 M 1/04, B 01 F 3/04

(21) Anmeldenummer: **79200562.1**

(22) Anmeldetag: **03.10.79**

(54) **Verfahren und Vorrichtung zum Begasen von Flüssigkeiten.**

(30) Priorität: **10.10.78 CH 10503/78**

(43) Veröffentlichungstag der Anmeldung: **16.04.80 (Patentblatt 80/08)**

(45) Bekanntmachung des Hinweises auf die Patenterteilung: **22.09.82 Patentblatt 82/38**

(84) Benannte Vertragsstaaten: **AT CH DE FR GB IT NL SE**

(56) Entgegenhaltungen:
**AU - 1344/66**
**FR - A - 2 162 684**
**GB - A - 1 048 905**
**US - A - 2 913 343**
**US - A - 3 017 951**

(73) Patentinhaber: **Chemap AG**
**Alte Landstrasse 415**
**CH-8708 Männedorf (CH)**

(72) Erfinder: **Müller, Hans, Dr. Ing.**
**Im Allmendli**
**CH-8703 Erlenbach (CH)**
Erfinder: **Müller, Felix, Dr.**
**Bahnhofstrasse 46**
**CH-8712 Stäfa (CH)**

(74) Vertreter: **Herrmann, Peter**
**Chemap AG Alte Landstrasse 415**
**CH-8708 Männedorf (CH)**

**EP 0 009 852 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren und Vorrichtung zum Begasen von Flüssigkeiten

Gegenstand der Erfindung ist ein Verfahren und eine Vorrichtung zur Begasung von Flüssigkeiten, insbesondere zur aeroben Züchtung von Mikroorganismen.

Begasungsvorrichtungen zur Züchtung von Mikroorganismen sind bekannt. Rührwerke mit Oben- oder Untenantrieb haben sich zur Umwälzung von Volumina bis etwa 300 $m^3$ als geeignet erwiesen. Zur Umwälzung grösserer Volumina in geschlossenen Behältern, wird Luft in den Boden des Behälters unter Druck eingeführt. Hierbei erfolgt eine Umwälzung des Behälterinhaltes nach dem Prinzip der Mammutpumpe.

Nachteile der bekannten Verfahren sind für grosse Volumina, dass bei mechanischer Rührung mit Rührwerken diese neben der grossen Energieaufnahme sehr lange Rührerwellen erfordern, wenn Obenantrieb vorgesehen wird und bei Untenantrieb Abdichtungsprobleme durch den hohen hydrostatischen Druck auftreten. Bei Einsatz von Fermentern nach dem Mammutpumpenprinzip sind Kompressoren von grossen Dimensionen nötig, um die erforderliche komprimierte Luft zu liefern. In keinem Fall jedoch ist bei grossen Volumina die erfoderliche Homogenität gewährleistet.

Nach der CH-A-572 978 ist eine Vorrichtung bekannt, die aus einem horizontal gelagerten Behälter besteht, der mehrere Umwälzeinrichtungen in Serie geschaltet aufweist. Jeder Block bildet eine Einheit in einem gemeinsamen Behälter.

Aus der AU 1344/66 ist zur Erzeugung einer spiralförmigen Strömung in einem offenen rechteckigen Abwasserbecken die Ueberlagerung einer Rotationsströmung in senkrechter Ebene mit einer Translationsströmung bekannt. Die Erzeugung der Rotationsströmung wird durch Flüssigkeitsstrahl über Düsen erzeugt.

Die US-A 3 017 951 beschreibt eine spiralförmige aufsteigende Bewegung von Luft und Abwasser in einem offenen rechteckigen Betonbecken.

Nach der GB-A 1 048 905 wird gleichfalls eine spiralförmige Rotationsströmung, die in senkrechter Ebene verläuft, in einem rechteckigen Abwasserbecken mit abgerundeten Kanten mittels eines Oberflächenbelüfters in Form rotierender Bürsten erzeugt.

Alle bekannten Begasungsvorrichtungen weisen den Nachteil auf, dass entweder an der nicht genau definierbaren Grenzfläche zwischen zwei benachbarten Begasungseinheiten oder in den Ecken der Becken Totzonen auftreten. Daneben ist auch die offene Bauweise für Fermentationen nicht geeignet.

Aufgabe der Erfindung ist es, ein Verfahren und eine Vorrichtung zur Begasung von Flüssigkeiten zu schaffen, die bei grossen Fermentern eine möglichst gute Homogenität des Behälterinhaltes gewährleisten.

Diese Aufgabe wird erfindungsgemäss durch ein Verfahren nach Anspruch 1 gelöst, das dadurch gekennzeichnet ist, dass die an den Hohlrührer austretende Flüssigkeit in zwei entgegengesetzte und zueinander versetzte Strömungen parallel zur Längsachse des Behälters geleitet wird und eine Vorrichtung, die dadurch gekennzeichnet ist, dass ein Leitapparat zur Erzeugung einer zur Längsachse des Behälters parallelen, in beiden Behälterhälften einander entgegengesetzten Strömung stationär um das Pumpenlaufrad angeordnet ist.

Durch das erfindungsgemässe Verfahren wird im Reaktor eine kontrollierbare Spiralströmung erzeugt, welche in sich geschlossen ist und eine optimale Begasung der Flüssigkeit und der zu kultivierenden Mikroorganismen bewirkt.

Die Erfindung soll anhand der Zeichnungen erläutert werden. Es zeigen

Figur 1 einen Längsschnitt durch den Behälter mit 3 baukastenartigen Belüftungseinheiten,

Figur 2 einen Querschnitt A-A durch den Behälter nach Fig. 1,

Firur 3 einen Querschnitt durch die Pumpeneinheit mit Leitapparat,

Figur 4 eine Draufsicht auf die Pumpeneinheit mit Leitapparat,

Figuren 5-7 Strömungsmuster.

Die Figur 1 zeigt in ihrer Gesamtheit einen liegenden Druckbehälter mit beispielsweise drei baukastenartigen Belüftungseinheiten 2, 2′ und 2″ mit beispielsweise gleichem Durchmesser und gleicher Länge. Der Kessel 1 ist an seinen beiden Enden 3 und 3′ halbkugelförmig verschlossen. Jede Einheit 2 kann eine Belüftungsvorrichtung 4, 4′ und 4″ aufweisen, die wiederum aus einem Leitrohr 5, einem internen Luftzufuhrrohr 6, einem Leitapparat 9, einer externen Luftzufuhr 7 und einem Pumpenrad 8 besteht.

Figur 2 zeigt den Querschnitt A-A der Figur 1 und lässt erkennen, dass Kühleinheiten 18 einschiebbar angeordnet sind.

Figur 3 zeigt das Pumpenlaufrad 8 mit Leitapparat 9 im Detail. Das Pumpenlaufrad 8 ist mit einer Welle 10, die mit einem Antrieb 19 verbunden ist, versehen. Auf seiner Oberseite weist das Pumpenlaufrad 8 eine Eintrittsöffnung 12 für die Flüssigkeit auf, die aus dem darüber befindlichen Leitrohr 5 angesaugt wird. Die Leiteinrichtung 9 ist in bekannter Weise am Boden des Kessels 1 befestigt und besteht aus einer Anzahl parallel gerichteter Rohre 13, deren Oeffnungen gemäss Figur 4 in entgegengesetzter Richtung weisen.

Figur 4 zeigt in Draufsicht wie der Leitapparat 9 mit dem Pumpenlaufrad 8 verbunden ist.

Figur 5 zeigt das Fliessmuster der einzelnen Strömungen. Durch den axialen Ansog über das Leitrohr 5 auf das Pumpenlaufrad 8 und die radiale Ausschleuderung wird zunächst eine Teilströmung 14 von oben nach unten erzeugt, die eine Umwälzung bewirkt. Durch die zahlreichen

gerichteten Rohre 13 des Leitapparates 9, deren Austritte in Figur 4 durch Pfeile angedeutet, jeweils gegenüberliegend angeordnet sind, wird eine laterale Strömung 15 erzeugt, die insgesamt in einer Spiralströmungsbewegung 16 gemäss Figuren 6 und 7 resultiert.

Figur 6 zeigt einen Aufriss;

Figur 7 den Grundriss durch den Behälter mit den jeweils zugeordneten Strömungsbildern.

Im Betrieb wird der Behälter zu etwa 2/3 mit Flüssigkeit oder flüssigem Nährsubstrat gefüllt. Beispielsweise bei gleichem Verhältnis von Durchmesser und Länge ergeben sich folgende Fühllhöhen:

| | |
|---|---|
| 50 $m^3$ | 2,261 m |
| 100 $m^3$ | 2,84 m |
| 200 $m^3$ | 3,58 m |

Die erforderlichen hydrostatischen Drücke können ohne weiteres noch mit einer selbstansaugenden Turbine beherrscht werden. Für beispielsweise einen Fermenter von 1 000 $m^3$ Totalvolumen sind 5 Einheiten an 200 $m^3$ erforderlich. Trotz des grossen Volumens beträgt dann die Füllhöhe nur 3,58 m. Das über einen nichtgezeigten, beispielsweise Elektromotor angetriebene Pumpenlaufrad 8 saugt Flüssigkeit über das Leitrohr 5 und gleichzeitig Luft aus dem Gasraum über das interne Belüftungsrohr 6 an. Diese interne Belüftung führt zu einer erhöhten Ausnutzung des Sauerstoffs in der Begasungsluft. Die Frischluftzufuhr kann in bekannter Weise über die hohl ausgebildete Antriebswelle 10 und/oder die Zuluftleitung 7 in den Leitapparat 9 erfolgen. Die Abluft verlässt über einen Stutzen 17 direkt oder unter Zwischenschaltung eines nicht gezeigten mechanischen Schaumabscheiders den Behälter 1. Die Anschlüsse für Zu- und Abfuhr der zu begasenden Flüssigkeit können an jeder beliebigen Stelle im unteren Teil des Behälters angebracht sein, wobei man vorzugsweise beide so weit wie möglich voneinander entfernt anbringt. Ueber den Leitapparat 9 wird Frischluft mit ca. 3 bar über Düsen in die engste Stelle der Venturiöffnung eingeleitet. Aus dem Fermenterkopfraum wird gleichzeitig Luft, welche mindestens noch 10% Sauerstoff enthält angesaugt. Dadurch wird eine Erhöhung der Sauerstoffausnutzung möglich.

## Ansprüche

1. Verfahren zur Begasung von Flüssigkeiten in einem geschlossenen, liegenden Behälter mit kreisförmigem Durchmesser mittels Hohlrührern, die in senkrechten Ebenen verlaufende Rotationsströmungen erzeugen, insbesondere zur aeroben Züchtung von Mikroorganismen, dadurch gekennzeichnet, dass die an den Hohlrührern austretende Flüssigkeit in zwei entgegengesetzte und zueinander versetzte Strömungen parallel zur Längsachse des Behälters geleitet wird.

2. Vorrichtung zur Durchführung des Verfahrens nach Anspruch 1, dadurch gekennzeichnet, dass ein Leitapparat (9) zur Erzeugung einer zur Längsachse des Behälters parallelen, in beiden Behälterhälften einander entgegengesetzten Strömung (15) stationär um das Pumpenlaufrad (8) angeordnet ist.

3. Vorrichtung nach Anspruch 2, bestehend aus einem Druckbehälter (1) mit einer oder mehreren Belüftungsvorrichtungen (4, 4', 4''), die aus Zentralrohr (5) und Pumpenlaufrad (8) bestehen, dadurch gekennzeichnet, dass der Behälter (1) aus einer oder mehreren baukastenartigen Belüftungseinheiten (2, 2', 2'') besteht und an den beiden Enden mit halbkugelförmigen Seitenteilen (3) versehen ist.

4. Vorrichtung nach Ansprüchen 2 und 3, dadurch gekennzeichnet, dass der Leitapparat (9) mit Anschlüssen (7) für die Zufuhr von Prozessluft ausgerüstet ist.

5. Vorrichtung nach Ansprüchen 2 bis 4, dadurch gekennzeichnet, dass die baukastenartigen Belüftungseinheiten (2, 2', 2'') das Verhältnis Durchmesser zu Länge 1 : 1 aufweisen.

## Claims

1. A process for gasification liquids, particularly for aerobic cultivation of micro-organisms inside a closed horizontal vessel of circular diameter, by means of hollow stirrers which create rotation currents, moving in directions rectangular to the axis of the stirrers, characterized in that the liquid which exits from the hollow stirrers is guided in two currents moving in opposite and mutually alternating directions, parallel to the longitudinal axis of the vessel.

2. An arrangement for carrying out the process according to claim 1, characterized in that a distributing element (9) is attached around the impeller (8) so that a stream (15) is created which moves parallel to the longitudinal axis of the vessel and in the two halves of the vessel in mutually opposite directions.

3. An arrangement as defined in claim 2 consisting of a pressure vessel (1) with one or several aeration units (4, 4', 4''), which consist of the central tube (5) and the impeller (8), characterized in that the vessel (1) consists of one or several modular aeration units (2, 2', 2'') and is provided at both ends with semispherical side pieces (3).

4. An arrangement according to claims 2 and 3, characterized in that the distributing element (9) is provided with connections (7) for the supply of air necessary for the process.

5. An arrangement according to claims 2-4, characterized in that the modular aeration units (2, 2', 2'') show the proportions of 1 : 1 for diameter and longitude.

## Revendications

1. Procédé pour l'introduction de gaz dans des liquides, dans un récipient cylindrique fermé,

horizontal, au moyen d'un agitateur tubulaire, notamment pour la culture aérobique de micro-organismes, caractérisé en ce que le flux sortant de l'agitateur est dirigé parallèlement à l'axe longitudinal du réservoir en deux courants étant opposés l'un à l'autre.

2. Dispositif pour la mise en œuvre du procédé selon la revendication 1, caractérisé en ce qu'un distributeur (9) est disposé stationnaire autour de la roue à aubes de pompage (8) pour créer, dans les deux moitiés du réservoir, un courant parallèle à l'axe longitudinal du réservoir, les deux courants étant opposés l'un à l'autre.

3. Dispositif selon la revendication 2, comprenant un réservoir sous pression (1) comprenant un ou plusieurs dispositifs d'aération (4, 4', 4''), constitués d'un tuyau central (5) et d'une roue à aubes de pompage (8), caractérisé en ce que le réservoir (1) est constitué d'une ou de plusieurs unités d'aération modulaires (2, 2', 2'') et comporte, aux deux extrémités, des éléments latéraux hémisphériques (3).

4. Dispositif selon les revendications 2 et 3, caractérisé en ce que le distributeur (9) est doté de raccords (7) pour l'admission de l'air du procédé.

5. Dispositif selon les revendications 2 à 4, caractérisé en ce que les unités d'aération modulaires (2, 2', 2'') présentent le rapport diamètre-longueur de 1 : 1.

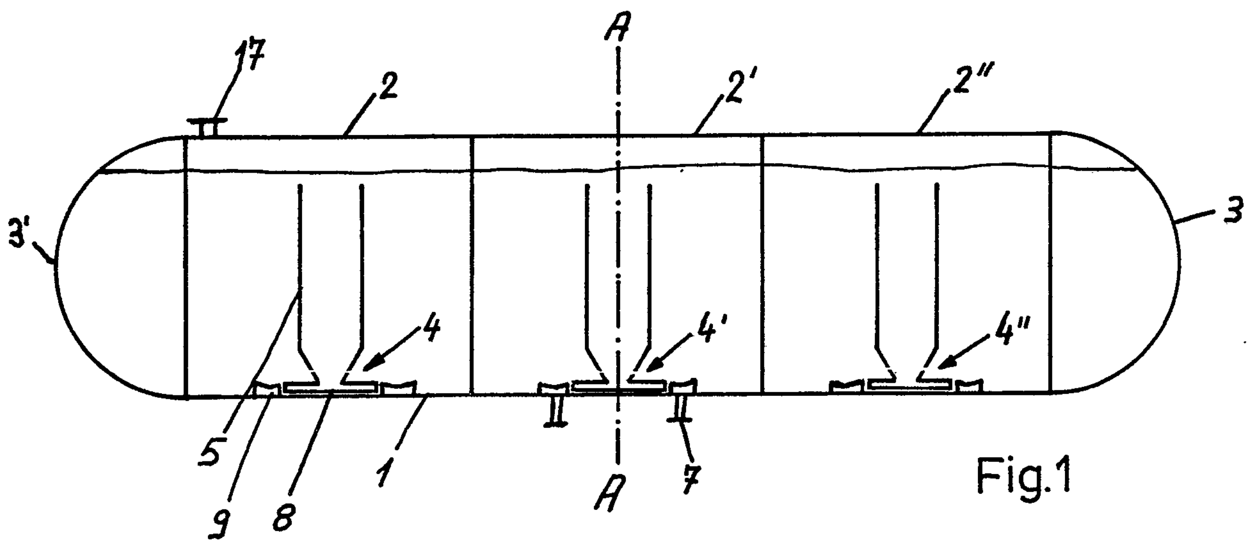


Fig.1

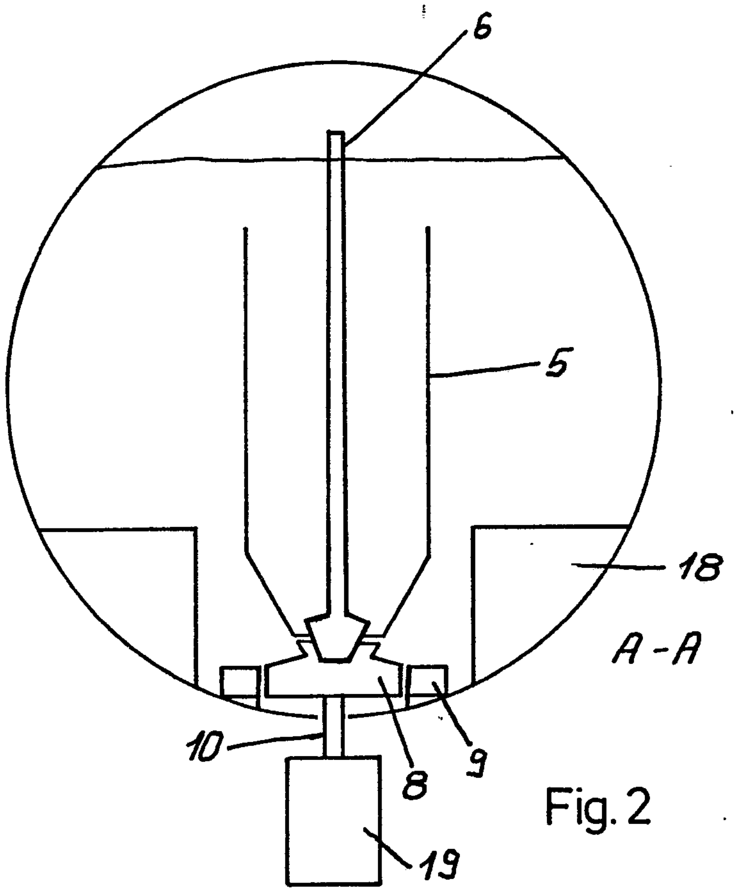


Fig. 2

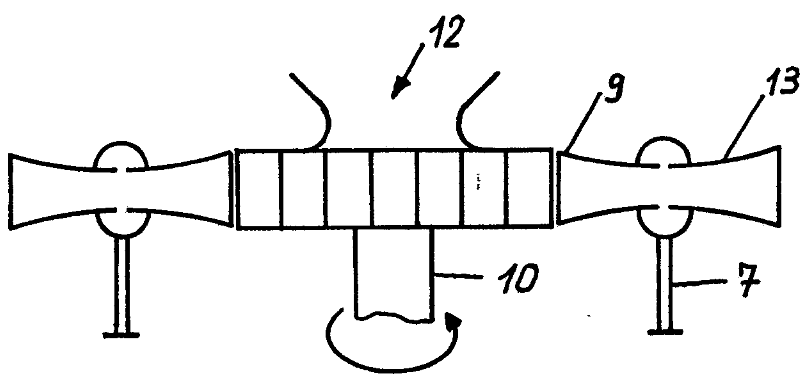


Fig.3

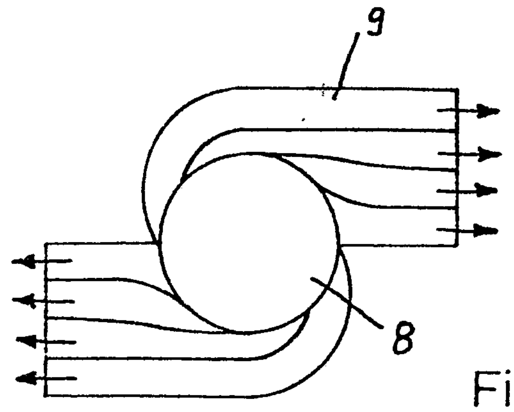


Fig. 4

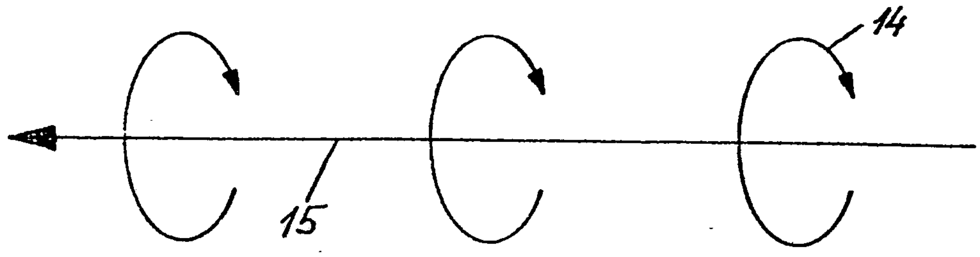


Fig. 5

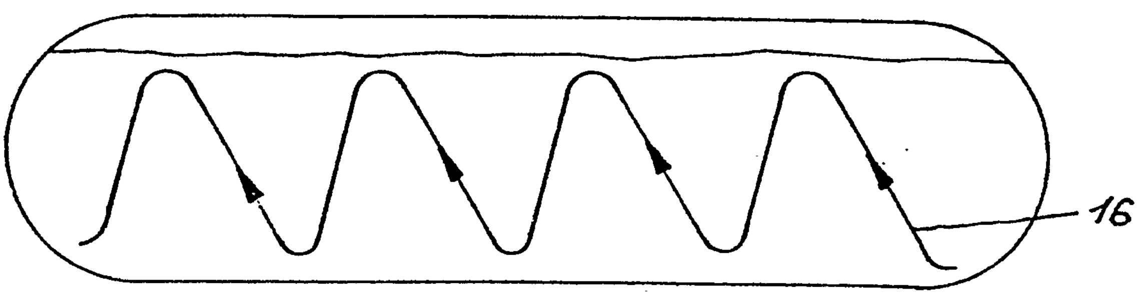


Fig. 6

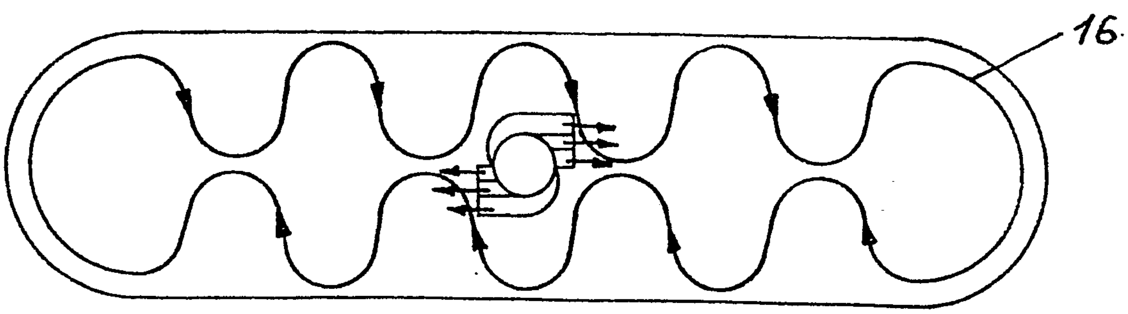


Fig. 7